# EUROPÄISCHE PATENTSCHRIFT

(11) **EP 2 760 436 B1**
(45) Veröffentlichungstag und Bekanntmachung des Hinweises auf die Patenterteilung: **18.09.2019**
(21) Anmeldenummer: 12762597.8
(22) Anmeldetag: 25.09.2012
(51) Int. Cl.: A61K 9/00, A61K 31/167, A61K 31/196, A61K 31/245, A61L 15/42, A61L 15/48, A61K 9/70, A61L 15/22, A61L 15/58, A61L 15/44

(54) **PFLASTER MIT EINSTELLBARER OKKLUSION**
PLASTER HAVING ADJUSTABLE OCCLUSION
PANSEMENTS À OCCLUSION AJUSTABLE

(30) Priorität: 26.09.2011 DE 102011114411
(43) Veröffentlichungstag der Anmeldung: 06.08.2014
(73) Patentinhaber: LTS LOHMANN Therapie-Systeme AG, 56626 Andernach (DE)
(72) Erfinder: MÜLLER, Walter, 56626 Andernach (DE); MOHR, Patrick, 53498 Bad Breisig (DE)
(74) Vertreter: Schweitzer, Klaus
(86) Internationale Anmeldenummer: PCT/EP2012/068828
(87) Internationale Veröffentlichungsnummer: WO 2013/045420

(56) Entgegenhaltungen:
- EP-A1- 0 379 045
- WO-A1-93/00058
- WO-A2-01/91718

## Beschreibung

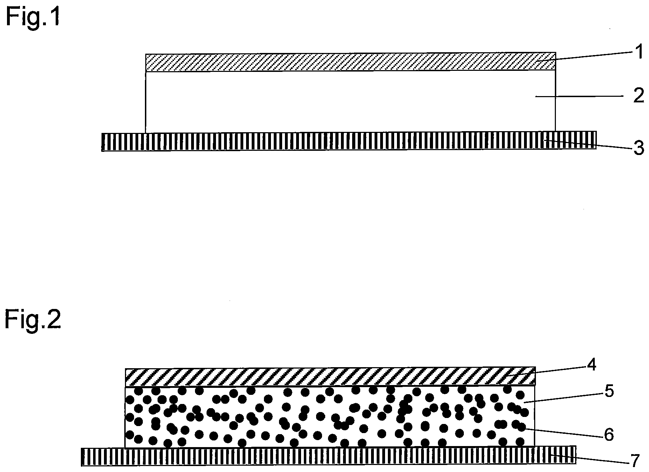

Bei der transdermalen oder topischen Verabreichung von Wirkstoffen ist das Stratum Corneum eine die Wirkstoffaufnahme begrenzende lipophile Barriere. Eine der wirksamsten und praktisch bei jedem systemisch wirkenden transdermalen System angewandte physikalische Methode zur Erniedrigung der Barrierefunktion des Stratum Corneums ist die Okklusion. Die Okklusion wird erreicht durch die Verwendung von praktisch wasserdampfundurchlässigen Materialien für die Rückschichten von transdermalen Systemen oder/und die Verwendung von wasserdampfundurchlässigen Formulierungen von angrenzenden Schichten bzw. der wirkstoffhaltigen Schicht oder der wirkstoffhaltigen Schichten. Eine schematische Darstellung eines solchen Pflasters zeigt Figur 1. Für die Rückschichten werden in den allermeisten Fällen Polyesterfolien verwendet. Grundsätzlich spricht allerdings nichts gegen andere Folien mit geringer Wasserdampfdurchlässigkeit wie z. B. Folien aus Polyethylen oder Polypropylen. Als Polymere oder Haftkleber mit geringer Wasserdampfdurchlässigkeit werden z. B. Polyisobutylen oder Blockpolymere aus Styrol und Butadien oder Styrol und Isopren verwendet.

Speziell topische Systeme mit z. B. nichtsteroidalen Antirheumatika haben eine Größe, die der Fläche des zu behandelnden Areals entspricht, und damit eine Größe, die zur Erhöhung des Tragekomforts eine gewisse Dehnbarkeit des Pflastersystems erfordert. Da die oben genannten okklusiven Materialien für die Rückschicht in der für diesen Zweck geeigneten Dicke über keine genügende Dehnbarkeit oder Elastizität verfügen, werden für solche Pflaster oft textile Materialien verwendet. Ein Nachteil dieser textilen Rückschichten ist allerdings, dass sie bedingt durch ihre Offenporigkeit eine sehr hohe Wasserdampfdurchlässigkeit besitzen und damit keine okklusiven Bedingungen schaffen. Daraus folgt, dass die Okklusion bei Pflastern mit textilen Rückschichten durch andere wasserdampfundurchlässige oder zumindest wenig wasserdampfdurchlässige sonstige Schichten erreicht werden muss. Naturgemäß können dafür keine nicht dehnbaren und unelastischen Materialien genommen werden. Am einfachsten erreicht man die Okklusion durch die Verwendung von wenig wasserdampfdurchlässigen Haftklebern wie Haftklebern auf Basis Polyisobutylen oder Blockpolymeren aus Styrol und Butadien oder Isopren. Nachteilig ist dabei jedoch, dass diese Haftkleber nur sehr schlecht auf der sich unter Okklusion durchfeuchtenden Haut haften und sich speziell im Gelenkbereich leicht teilweise oder vollständig ablösen.

In der EP 0 379 045 A1 ist eine Zusammensetzung für ein transdermales System offenbart, die neben einem pharmazeutischen Wirkstoff ein Ethylen/Vinylacetat (EVA)-Copolymer und ein Acrylatpolymer, einen Gummi und ein Haftmittel umfaßt. Das Gewichtsverhältnis von Ethylen/Vinylacetat-Copolymer zu dem Acrylatpolymer liegt dabei vorzugsweise im Bereich von 20 : 1 bis 1 : 20, während das Verhältnis von EVA-Copolymer und Acrylatpolymer zu dem Gummi vorzugsweise im Bereich von 1 : 10 bis 30 : 1 liegt. Das Gummi ist bevorzugt ein natürliches oder synthetisches Polyisopren, ein Styrol-Isopren-Styrol-Blockcopolymer, Polybutylen oder Polyisobutylen. Schichten aus der Zusammensetzung zeigen bei Applikation auf der Haut zufriedenstellende Haft- und Ablöseeigenschaften über mehrere Tage. Das gilt auch dann, wenn die Schicht einen pharmazeutischen Wirkstoff mit weichmachenden Eigenschaften, beispielsweise Nitroglycerin, in einem höheren Anteil enthält.

Gegenstand der WO 93/00058 ist ein transdermales therapeutisches System mit einer wirkstoffhaltigen Matrixschicht, die zwei verschiedene Haftkleber enthält. Der erste Haftkleber ist bevorzugt ein Polyacrylat, der zweite ein Polysiloxan. Offenbart sind auch Matrixschichten, die 33,1 Gew.-% an Polyacrylat und 40,1 Gew.-% an Polyisobutylen bzw. jeweils 45 Gew.-% an Polyacrylat und Polyisobutylen enthalten.

In der WO 01/91718 A2 ist eine zweiphasige Matrix beschrieben, bei der eine wirkstoffhaltige Polyacrylatphase in einer äußeren, selbstklebenden Formulierung auf Basis von Polyisobutylen oder Styrol-Butadien-Styrol-Blockpolymeren dispergiert ist. Nachteilig ist hier, dass der Okklusionseffekt bei praktischen Schichtdicken immer maximal ist und die äußere Phase, wie schon oben ausgesagt, auf feuchter Haut sehr schlecht klebt. Der einzige Vorteil einer solchen Matrix ist, dass sich der Wirkstoff in einem Polymer mit einer höheren Sättigungslöslichkeit befindet.

Polyacrylatkleber oder Silikonkleber verhalten sich unter solchen Bedingungen wesentlich besser, können allerdings durch ihre hohe Wasserdampfdurchlässigkeit selbst keine okklusiven Bedingungen schaffen. Die Verwendung von Polyacrylat- oder Silikonklebern würde deshalb gemäß dem Stand der Technik mehrschichtige Matrices mit Schichten unterschiedlicher Zusammensetzung notwendig machen, damit das Herstellverfahren verkomplizieren und den bei dieser Produktgruppe wichtigen Herstellpreis in die Höhe treiben.

Es besteht deshalb das Bedürfnis nach okklusiven Pflastern mit einer textilen, dehnbaren Rückschicht und auf feuchter Haut gut haftenden Haftklebern auf Basis von Polyacrylaten oder Silikonklebern, die über einen möglichst einfachen Aufbau verfügen und einfach herzustellen sind.

Überraschenderweise wurde die Lösung des Problems erfindungsgemäß darin gefunden, ein wenig wasserdampfdurchlässiges Polymer wie z.B. Polyisobutylen oder ein Blockpolymer aus Styrol und Butadien oder Isopren in der oder den aus wasserdampfdurchlässigen Haftklebern aufgebauten Matrixschicht(en) zu dispergieren. Die Wirkstoffe sind durch ihre physikochemischen Eigenschaften bedingt dabei zum allergrößten Teil in der Polyacrylatphase einer solchen Matrix enthalten. Durch die Menge an wasserdampfundurchlässigem Polymer und der Dicke der Matrix lässt sich der Okklusionseffekt in weiten Grenzen variieren. Im einfachsten Fall besteht damit ein erfindungsgemäßes Pflaster aus einer textilen Rückschicht, einer wirkstoffhaltigen Matrixschicht auf Basis von Polyacrylat- oder Silikonklebern mit darin dispergiertem wasserdampfundurchlässigem Polymer und einer vor Gebrauch zu entfernenden Schutzschicht. Der Aufbau eines solches Pflasters ist in Figur 2 dargestellt.

Die Erfindung betrifft daher ein transdermales oder topisches, wirkstoffhaltiges Pflaster mit einer nichtokklusiven Rückschicht, die aus einem textilen Material besteht, einer Matrix, gebildet aus einer oder mehreren Polymerschicht(en) mit mindestens einem pharmazeutischen Wirkstoff in einer oder mehreren der Schichten, das dadurch gekennzeichnet ist, dass die strukturbildenden Basispolymere der Schicht oder der Schichten der bei Anwendung mit der Haut in Kontakt kommenden Matrixschicht nicht oder nur wenig okklusiv und Haftkleber sind, wobei die Haftkleber Polyacrylatkleber oder Silikonkleber sind, und in zumindest einer der Polymerschichten ein mit dem Basispolymer nicht oder nur sehr wenig mischbares zweites Polymer mit einer geringen Wasserdampfdurchlässigkeit, welches Polyisobutylen, ein Styrol-Isopren-Styrol-Blockcopolymer oder ein Styrol-Butadien-Styrol-Blockcopolymer ist, dispergiert ist, wobei der Anteil an dispergierten Partikeln des zweiten Polymers in der Matrix zwischen 10 und 30 Gew.-% liegt und wobei das Flächengewicht der Matrix zwischen 50 und 400 g/m² liegt.

Die Rückschicht besteht aus einem textilen Material, insbesondere einem Gewebe oder nicht-gewebten Vliesstoff bzw. einem Verbund aus solchen. Als Materialien kommen hier z. B. Baumwolle, Viskose, Polyester, Polyamide, Polyurethan oder Polypropylen in Frage.

Das strukturbildende Basispolymer der bei Anwendung mit der Haut in Kontakt kommenden Matrixschicht ist ein Polyacrylatkleber oder Silikonkleber. Die Matrix ist vorzugsweise einschichtig aufgebaut.

Das Polymer mit geringer Wasserdampfdurchlässigkeit ist Polyisobutylen, ein Styrol-Isopren-Styrol-Blockpolymer oder ein Styrol-Butadien-Styrol-Blockpolymer. Die dispergierte Phase dieses Polymers hat vorzugsweise eine mittlere Teilchengröße von 5 bis 50 µm, insbesondere 7 bis 40 µm, ganz besonders bevorzugt 10 bis 30 µm. Der Anteil der dispergierten Partikel in der Matrix liegt zwischen 10 und 30 Gew.-%.

Das Flächengewicht der Matrix liegt zwischen 50 und 400 g/m², vorzugsweise zwischen 60 und 300 g/m², insbesondere zwischen 70 und 200 g/m². Der pharmazeutische Wirkstoff kann ein nichtsteroidales Antirheumatikum (dt. NSAR für nicht-steriodales Antirheumatikum, engl. NSAID für non-steriodal anti inflammatory drug) sein. Diese werden häufig lokal äußerlich angewendet im Bereich von Gelenken, insbesondere der Extremitäten. Gerade an diesen mechanisch stark beanspruchten Applikationsorten erweisen sich die erfindungsgemäßen TTS als besonders vorteilhaft. Ohne Anspruch auf Vollständigkeit handelt es sich um Wirkstoffe aus der Gruppe von Diclofenac oder eines seiner pharmazeutisch akzeptablen Salze, Ketoprofen, Ibuprofen, Flurbiprofen, Naproxen, Tiaprofensäure, Indomethacin, Piroxicam, Tenoxicam, Meloxicam, Flufenaminsäure oder Mefenaminsäure. Bevorzugte Diclofenacsalze sind beipielsweise das Diclofenac-Natriumsalz, das Diclofenac-Kaliumsalz, das Diclofenac-Diethylammoniumsalz oder das Dihydroxyethyl-pyrrolidinsalz von Diclofenac.

Weiterhin kommen als Wirkstoffe topisch wirksame Analgetika, z. B. Lidocain oder Tetracain, in Frage.

Neben den bereits erwähnten Polymeren und Wirkstoffen können noch zahlreiche andere Hilfsstoffe zum Einsatz kommen, wie sie für die Verwendung in TTS der Fachwelt bekannt sind.

So können z. B. Permeationsverstärker, vorzugsweise in der inneren Phase der Matrix, eingesetzt werden. Als Permeationsverstärker kommen in Frage Verbindungen aus der Gruppe der niedermolekularen ein- oder mehrwertigen Alkohole, Fettsäuren (vorzugsweise Ölsäure), Fettalkohole, Fettalkoholether, polyoxyethylierte Fettalkohole, Fettsäureester (speziell Monoglyceride und Monoester mit Propylenglykol), Sorbitanfettsäureester und polyoxyethylierte Sorbitanfettsäurester sowie Dimethylisosorbit.

Ferner kommen in Frage grenzflächenaktive Tenside, die die Stabilität der zweiphasigen Matrixschicht positiv zu beeinflussen vermögen, indem sie die Grenzflächenenergie herabsetzen.
Das wasserdampfundurchlässige Polymer ist in die Matrix eingebettet. Es kommt deshalb praktisch nicht in Kontakt mit der Haut und beeinträchtigt deshalb nicht das Haftvermögen des Haftklebers auf der Haut.

Die Verringerung der Wasserdampfdurchlässigkeit beruht dabei auf der Verlängerung des effektiven Diffusionsweges der Wassermoleküle. Dies bedeutet auch, dass die Größe des Effekts abhängt von der Menge des dispergierten Polymers und natürlich von der Gesamtdicke der Matrixschicht.

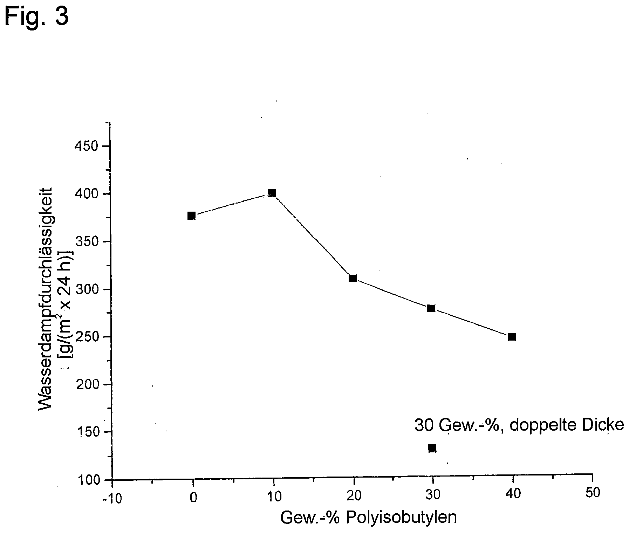

Dieser Zusammenhang wurde an einem Polyacrylatkleber und niedermolekularem Polyisobutylen experimentell untersucht. Dazu wurden Kleberfilme unterschiedlicher Dicke und unterschiedlichen Polyisobutylengehalts hergestellt und die Wasserdampfdurchlässigkeit nach der DIN-Methode EN 13726-2 bei einer Probengröße von 20 cm² bei 37 °C und 18 % rel. Feuchte gemessen. Die Probenzusammensetzung und die gemessene Wasserdampfdurchlässigkeit sind in Tabelle 1 und die Wasserdampfdurchlässigkeit zusätzlich in Figur 3 dargestellt.
Die Wasserdampfdurchlässigkeit liegt dann in der Regel zwischen 50 und 600 g/(m² x 24 h), vorzugsweise zwischen 100 und 500 g/(m² x 24 h), insbesondere zwischen 150 und 400 g/(m² x 24 h).

**Tabelle 1: Probenzusammensetzung und Wasserdampfdurchlässigkeit (die Zusammensetzungen in der ersten und in der letzten Zeile sind nicht gemäß der vorliegenden Erfindung)**

| Anteil an Polyacrylatkleber¹⁾ [Gew.-%] | Anteil an Polyisobutylen ²⁾ [Gew.-%] | Flächengewicht [g/m²] | Wasserdampfdurchlässigkeit [g/(m² x 24 h)] |
|---|---|---|---|
| 100 | 0 | 106 | 376 |
| 90 | 10 | 100 | 399 |
| 80 | 20 | 97 | 308 |
| 70 | 30 | 95 | 276 |
| 70 | 30 | 190 | 129 |
| 60 | 40 | 99 | 245 |

| | | | |
|---|---|---|---|
| 1) Duro-Tak® 387-2353, Henkel 2) Oppanol® B 10, BASF | | | |

Wie man erkennt, haben 10 Gew.-% Polyisobutylen noch praktisch keinen Effekt, 20 Gew.-% schon einen deutlichen Effekt, und bei 40 Gew.-% ist die Durchlässigkeit fast halbiert. Wie erwartet hängt die Durchlässigkeit auch von der Dicke der Schicht ab, d. h. die Durchlässigkeit wird durch eine Verdopplung halbiert.

Des weiteren wurde der Einfluss des Polyisobutylengehalts auf die Permeationsrate aus einem Pflastersystem untersucht. Als Wirkstoff wurde dazu Diclofenac-Natriumsalz gewählt und in ein einschichtiges Matrixsystem mit textiler Rückschicht eingearbeitet. Unter der Annahme, dass das hydrophile Wirkstoffsalz nur in vernachlässigbarer Menge in Polyisobutylen löslich ist, wurde die Wirkstoffmenge so gewählt, dass die Wirkstoffkonzentration trotz unterschiedlichen Polyisobutylengehalts in der äußeren Polyacrylatphase in allen Proben gleich blieb. Damit wurde ausgeschlossen, dass Unterschiede in den Permeationsraten nicht nur auf einer verschieden starken Okklusion, sondern auch auf unterschiedlichen thermodynamischen Aktivitäten beruhen.

Die Permeationsstudien wurden unter Verwendung der dem Fachmann gut bekannten Franz-Diffusionszellen und menschlicher Epidermis durchgeführt. Die Werte in den Tabellen sind die Mittelwerte aus jeweils 4 unabhängigen Experimenten.

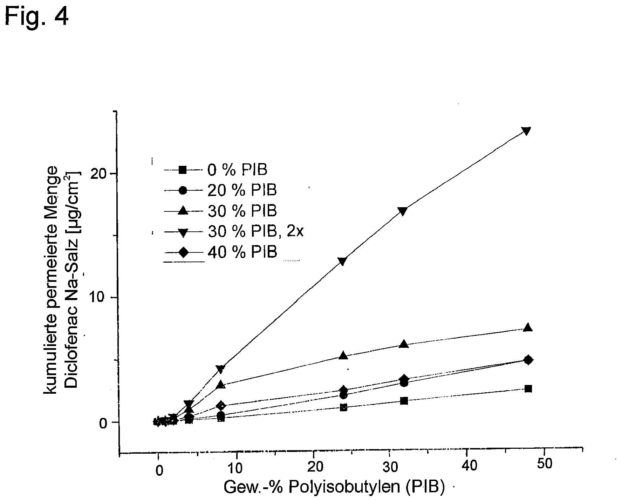

Die Zusammensetzung der Proben und die dazugehörigen Permeationsraten sind in den Tabellen 2 und 3 zusammengefasst und die Permeationsraten zusätzlich in Figur 4 graphisch dargestellt.

**Tabelle 2: Zusammensetzung der Proben für Permeationsstudien (die Proben 1 und 5 sind nicht gemäss der vorliegenden Erfindung)**

| Probennummer | Gew.-Teile Polyacrylatkleber Duro-Tak® 387-2353 | Gew.-Teile Polyisobutylen Oppanol® B 10 | Gew.-Teile Diclofenac-Na- Salz | Gew.-Teile Ölsäure | Flächengewicht [g/ m²] |
|---|---|---|---|---|---|
| 1 | 84 | 0 | 6 | 10 | 100±5 |
| 2 | 84 | 20 | 6 | 10 | 100±5 |
| 3 | 84 | 30 | 6 | 10 | 100±5 |
| 4 ¹⁾ | 84 | 30 | 6 | 10 | 200±5 |
| 5 | 84 | 40 | 6 | 10 | 100±5 |

| | | | | | |
|---|---|---|---|---|---|
| 1) doppelte Schichtdicke wie Probe 3 | | | | | |

**Tabelle 3: Permeationsraten unter Verwendung von menschlicher Epidermis**

| | Permeationsdauer und permeierte Menge Diclofenac Na-Salz [µg/ cm²] | | | | | | |
|---|---|---|---|---|---|---|---|
| Probennummer | 1 h | 2 h | 4 h | 8 h | 24 h | 32 h | 48 h |
| 1 | 0 | 0,023 | 0,083 | 0,219 | 0,98 | 1,45 | 2,32 |
| 2 | 0 | 0,05 | 0,16 | 0,40 | 1,82 | 2,85 | 4,65 |
| 3 | 0,03 | 0,20 | 0,93 | 2,85 | 5,10 | 5,93 | 7,17 |
| 4 ¹⁾ | 0,08 | 0,37 | 1,42 | 4,22 | 12,8 | 16,8 | 23,1 |
| 5 | 0 | 0,10 | 0,37 j | 1,22 | 2,34 | 3,19 | 4,64 |

| | | | | | | | |
|---|---|---|---|---|---|---|---|
| 1) doppelte Schichtdicke wie Probe 3 | | | | | | | |

Die Ergebnisse der Permeationsstudie zeigen klar, dass die Permeationsrate vom Gehalt an Polyisobutylen und der dadurch erhöhten Okklusion abhängt. Eine andere Erklärung kommt nicht in Frage, da die Zusammensetzung der wirkstoffhaltigen Phase in allen Proben gleich ist und der Zusatz von Polyisobutylen sogar den relativen Anteil dieser Phase in der Matrix verkleinert. Allerdings scheint das Optimum des Polyisobutylengehalts bei 30 Gew.-% zu liegen, da 40 Gew.-% in etwa das gleiche Ergebnis wie 20 Gew.-% zeigen. Bei höheren Gehalten wirkt sich dann der kleiner werdende Anteil der wirkstoffhaltigen Phase und der auch für den Wirkstoff länger werdende effektive Diffusionsweg nachteilig aus.

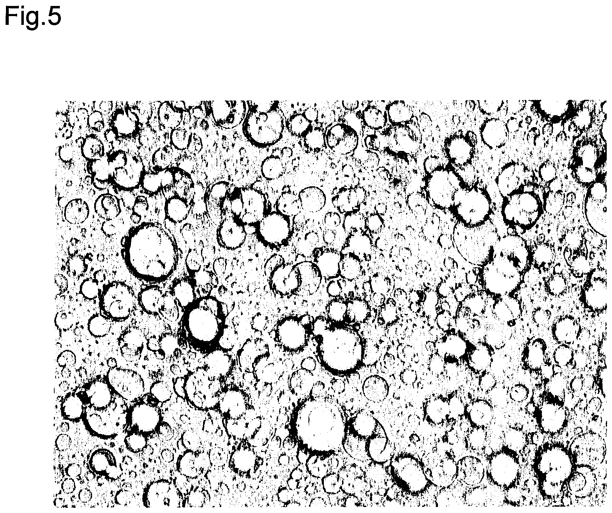

Da die innere Phase von der äußeren Phase, wie in Figur 5 gezeigt, getrennt ist und keine Mischung auf molekularer Ebene vorliegt, beeinflusst die Zugabe von innerer Phase mit geringer Wasserdampfdurchlässigkeit nicht die Sättigungslöslichkeit der Wirkstoffe in der aus Polyacrylaten oder Silikonen bestehenden äußeren Phase. Dies bedeutet, dass in solchen Systemen die Wirkstoffabgabe nicht durch eine Änderung der Sättigungslöslichkeit in der wirkstoffhaltigen Phase, wie z. B. in der beispielhaft genannten US-Patentschrift 6,235,306, beeinflusst wird. In den in Tabelle 2 genannten und in den Permeationsstudien, Tabelle 3, eingesetzten Formulierungen ist das Verhältnis von Acrylatkleber und Wirkstoff gleichgehalten, d. h. die Unterschiede in den gemessenen Permeationsraten können nur auf einer mit der Menge an Polyisobutylen ansteigenden Okklusion erklärt werden.

Die nachstehenden Herstellungsbeispiele dienen zur Illustration der Erfindung, ohne dass diese darauf beschränkt wäre.

### Beispiel 1

Herstellung von Pflastern mit Diclofenac-Natriumsalz gemäß Formulierung 1, 2, 3, 4 und 5, wobei Formulierungen 1 und 5 nicht gemäß der vorliegenden Erfindung sind. 90 g Polyisobutylen (Oppanol® B 10, BASF) werden in 110 g n-Heptan durch Rühren gelöst. Es resultieren 200 g Polyisobutylenlösung mit einem Feststoffgehalt von 45 %g/g.
20 g Diclofenac-Natriumsalz werden unter Rühren in 774 g Duro-Tak® 387-2353 (Feststoffgehalt 36 %), 150 g Ethylacetat und 33 g Ölsäure gelöst. Es resultieren 929 g wirkstoffhaltige Polyacrylatlösung mit einem Feststoffgehalt von 34 %g/g.

Die Beschichtungsmasse wird hergestellt durch Zugabe der in Tabelle 4 angegebenen Mengen Polyisobutylenlösung zu jeweils 100 g der wirkstoffhaltigen Polyacrylatlösung.

**Tabelle 4: Zusammensetzung der Formulierungen 1 bis 5**

| Formulierung | Polyisobutylen in Matrix [%g/g] | Zuwaage Polyisobutylenlösung [g] |
|---|---|---|
| 1 | 0 | 0 |
| 2 | 20 | 15,96 |
| 3,4 | 30 | 23,91 |
| 5 | 40 | 31,91 |

Die Dispersionen werden hergestellt durch 10-minütiges schnelles mechanisches Rühren mit 400 U/min. Die Matrices werden hergestellt durch Beschichten dieser Dispersionen auf eine silikonisierte 100 µm dicke Polyesterfolie mit anschließender Entfernung der Lösemittel durch 25-minütige Trocknung bei 50 °C. Die Dicke des noch lösemittelhaltigen Beschichtungsfilms wird so gewählt, dass der trockene Matrixfilm ein Flächengewicht von 110 g/ m² hat. Die Matrix zu Formulierung 4 wird erhalten, indem der schon getrocknete Matrixfilm zu Formulierung 3 einmal mit sich selbst laminiert wird.

Die getrockneten Filme werden mit einem bielastischen Polyestergewebe kaschiert und so das Gesamtlaminat erhalten.

Die fertigen Pflaster bzw. die Proben für die Permeationsstudien werden aus dem Gesamtlaminat gestanzt.

### Beispiel 2

Herstellung eines lidocainhaltigen Pflasters
50 g Lidocain werden in
1164 g Polyacrylatlösung (Duro-Tak® 387-2052, Henkel, Feststoffgehalt 47 %g/g
74 g Ethanol
110 g Ethylacetat
6 g Menthol
100 g Ölsäure
unter Rühren gelöst. In dieser Lösung wird 631 g einer Polyisobutylenlösung (48 %g/g in n-Heptan) dispergiert. Die Masse wird in einer Dicke auf eine silikonisierte Polyesterfolie beschichtet, dass nach dem Entfernen der Lösemittel (10 Minuten bei Raumtemperatur, 25 Minuten bei 50 °C) ein Flächengewicht von 135 g/ m² resultiert. Der getrocknete Film wird mit einem bielastischen Polyestergewebe kaschiert und so das Gesamtlaminat erhalten.

### Kurze Beschreibung der Abbildungen und der Bezugszeichen

Fig. 1: Okklusives Pflaster mit wasserdampfundurchlässiger Rückschicht
   1 wasserdampfundurchlässige Rückschicht
   2 wirkstoffhaltige Matrix
   3 wiederablösbare Schutzfolie
Fig. 2: Erfindungsgemäßes Pflaster mit einstellbarer Okklusion
   4 Rückschicht aus textilem Material
   5 strukturbildendes Basispolymer
   6 Partikel des Polymers mit geringerer Wasserdampfdurchlässigkeit
   7 wiederablösbare Schutzfolie
Fig. 3: Wasserdampfdurchlässigkeit als Funktion des Gehalts an Polyisobutylen und der Dicke der Matrixschicht
Fig. 4: Anteil Polyisobutylen und kumuliert permeierte Menge Diclofenac Na-Salz
Fig. 5: In der Polyacrylatkleberphase dispergierte Polyisobutylenpartikel (Maßstab: 500:1)

## Patentansprüche

1. Transdermales oder topisches wirkstoffhaltiges Pflaster mit einer nichtokklusiven Rückschicht, die aus einem textilen Material besteht, einer Matrix gebildet aus einer oder mehreren Polymerschicht(en) mit mindestens einem pharmazeutischen Wirkstoff in einer oder mehreren der Schichten, **dadurch gekennzeichnet, dass** die strukturbildenden Basispolymere der Schicht oder der Schichten der bei Anwendung mit der Haut in Kontakt kommenden Matrixschicht nicht oder nur wenig okklusiv und Haftkleber sind, wobei die Haftkleber Polyacrylatkleber oder Silikonkleber sind und in zumindest einer der Polymerschichten ein mit dem Basispolymer nicht oder nur sehr wenig mischbares zweites Polymer mit einer geringen Wasserdampfdurchlässigkeit, welches Polyisobutylen, ein Styrol-Isopren-Styrol-Blockpolymer oder ein Styrol-Butadien-Styrol-Blockpolymer ist, dispergiert ist, wobei der Anteil an dispergierten Partikeln des zweiten Polymers in der Matrix zwischen 10 und 30 Gew.-% liegt, wobei das Flächengewicht der Matrix zwischen 50 und 400 g/m² liegt.

2. Transdermales oder topisches Pflaster gemäß Anspruch 1, **dadurch gekennzeichnet, dass** die Matrix einschichtig ist.

3. Transdermales oder topisches Pflaster gemäß Anspruch 1, **dadurch gekennzeichnet, dass** die im Basispolymer dispergierte Phase des Polymers mit geringer Wasserdampfdurchlässigkeit eine mittlere Teilchengröße von 5 bis 50 µm, vorzugsweise 7 bis 40 µm, insbesondere 10 bis 30 µm hat.

4. Transdermales oder topisches Pflaster gemäß einem oder mehrerer der Ansprüche 1 bis 3, **dadurch gekennzeichnet, dass** das Flächengewicht der Matrix zwischen 60 und 300 g/m², insbesondere zwischen 70 und 200 g/m² liegt.

5. Transdermales oder topisches Pflaster gemäß einem oder mehrerer der Ansprüche 1 bis 4, **dadurch gekennzeichnet, dass** dessen Wasserdampfdurchlässigkeit gemessen nach der DIN-Methode EN 13726-2 bei einer Probengröße von 20 cm² bei 37 °C und 18 % relativer Feuchte zwischen 50 und 600 g/(m² x 24 h), vorzugsweise zwischen 100 und 500 g/(m² x 24 h), insbesondere zwischen 150 und 400 g/(m² x 24 h) liegt.

6. Transdermales oder topisches Pflaster gemäß einem oder mehreren der Ansprüche 1 bis 5, **dadurch gekennzeichnet, dass** der pharmazeutische Wirkstoff ein nichtsteroidales Antirheumatikum ist.

7. Transdermales oder topisches Pflaster gemäß Anspruch 6, **dadurch gekennzeichnet, dass** der pharmazeutische Wirkstoff Diclofenac oder eines seiner pharmazeutisch akzeptablen Salze, Ketoprofen, Ibuprofen, Flurbiprofen, Naproxen, Tiaprofensäure, Indomethacin, Piroxicam, Tenoxicam, Meloxicam, Flufenaminsäure oder Mefenaminsäure ist.

8. Transdermales oder topisches Pflaster gemäß Anspruch 7, **dadurch gekennzeichnet, dass** der pharmazeutische Wirkstoff ein Diclofenacsalz, insbesondere Diclofenac-Natriumsalz, Diclofenac-Kaliumsalz, Diclofenac-Diethylammoniumsalz oder das Dihydroxyethyl-pyrrolidinsalz von Diclofenac ist.

9. Transdermales oder topisches Pflaster gemäß einem oder mehreren der vorangegangenen Ansprüche 1 bis 5, **dadurch gekennzeichnet, dass** der pharmazeutische Wirkstoff ein topisch wirksames Analgetikum ist.

10. Transdermales oder topisches Pflaster gemäß Anspruch 9, **dadurch gekennzeichnet, dass** das topisch wirksame Analgetikum Lidocain oder Tetracain ist.

11. Transdermales oder topisches Pflaster gemäß einem oder mehreren der Ansprüche 1 bis 10, **dadurch gekennzeichnet, dass** die Matrix mindestens einen Permeationsverstärker, vorzugsweise im strukturbildenden Basispolymer, enthält.

12. Transdermales oder topisches Pflaster gemäß Anspruch 11, **dadurch gekennzeichnet, dass** der oder die Permeationsverstärker niedermolekulare ein- oder mehrwertige Alkohole, Fettsäuren, vorzugsweise Ölsäure, Fettalkohole, Fettalkoholether, polyoxyethylierte Fettalkohole, Fettsäureester, Sorbitanfettsäureester, polyoxyethylierte Sorbitanfettsäurester und/oder Dimethylisosorbit sind.

## Claims

1. Transdermal or topical active ingredient patch having a nonocclusive backing layer which consists of a textile material, a matrix formed from one or more polymer layers, with at least one active pharmaceutical ingredient in one or more of the layers, **characterized in that** the structure-forming base polymers of the layer or layers of the matrix layer, which comes into contact with the skin on application, are nonocclusive or minimally occlusive and are pressure-sensitive adhesives, wherein the pressure-sensitive adhesives are polyacrylate adhesives or silicone adhesives and in at least one of the polymer layers there is a second polymer dispersed, which is immiscible or very minimally miscible with the base polymer and has a low water vapor permeability, which is polyisobutylene, a styrene-isoprene-styrene block polymer or a styrenebutadiene-styrene block polymer, the fraction of dispersed particles of the second polymer in the matrix being between 10 and 30 wt %, wherein the basis weight of the matrix is between 50 and 400 g/m².

2. Transdermal or topical patch according to Claim 1, **characterized in that** the matrix is single-layer.

3. Transdermal or topical patch according to Claim 1, **characterized in that** the phase of the polymer with low water vapor permeability, dispersed in the base polymer, has an average particle size of 5 to 50 µm, preferably 7 to 40 µm, more particularly 10 to 30 µm.

4. Transdermal or topical patch according to one or more of Claims 1 to 3, **characterized in that** the basis weight of the matrix is between 60 and 300 g/m², more particularly between 70 and 200 g/m².

5. Transdermal or topical patch according to one or more of Claims 1 to 4, **characterized in that** its water vapor permeability as measured by the DIN method EN 13726-2 for a sample size of 20 cm² at 37 °C and 18 % relative humidity is between 50 and 600 g/(m² × 24 h), preferably between 100 and 500 g/(m² × 24 h), more particularly between 150 and 400 g/(m² × 24 h).

6. Transdermal or topical patch according to one or more of Claims 1 to 5, **characterized in that** the active pharmaceutical ingredient is a nonsteroidal antiinflammatory drug.

7. Transdermal or topical patch according to Claim 6, **characterized in that** the active pharmaceutical ingredient is diclofenac or a pharmaceutically acceptable salt thereof, ketoprofen, ibuprofen, flurbiprofen, naproxen, tiaprofenic acid, indomethacin, piroxicam, tenoxicam, meloxicam, flufenaminic acid, or mefenaminic acid.

8. Transdermal or topical patch according to Claim 7, **characterized in that** the active pharmaceutical ingredient is a diclofenac salt, more particularly diclofenac sodium salt, diclofenac potassium salt, diclofenac diethylammonium salt, or the dihydroxyethylpyrrolidine salt of diclofenac.

9. Transdermal or topical patch according to one or more of preceding Claims 1 to 5, **characterized in that** the active pharmaceutical ingredient is a topically active analgesic.

10. Transdermal or topical patch according to Claim 9, **characterized in that** the topically active analgesic is lidocaine or tetracaine.

11. Transdermal or topical patch according to one or more of Claims 1 to 10, **characterized in that** the matrix comprises at least one permeation enhancer, preferably in the structure-forming base polymer.

12. Transdermal or topical patch according to Claim 11, **characterized in that** the permeation enhancer or enhancers are low molecular mass, monohydric or polyhydric alcohols, fatty acids, preferably oleic acid, fatty alcohols, fatty alcohol ethers, polyoxyethylated fatty alcohols, fatty acid esters, sorbitan fatty acid esters, polyoxyethylated sorbitan fatty acid esters, and/or dimethylisosorbitol.

## Revendications

1. Pansement transdermique ou topique contenant un principe actif avec une couche arrière non occlusive, qui est constituée d'un matériau textile, une matrice formée d'une ou plusieurs couche(s) polymère(s) avec au moins un principe actif pharmaceutique dans une ou plusieurs des couches, **caractérisé en ce que** les polymères de base formant la structure de la couche ou des couches de la couche de matrice venant en contact avec la peau lors d'une utilisation ne sont pas ou ne sont que peu occlusifs et sont des adhésifs de contact, dans lequel les adhésifs de contact sont des adhésifs en polyacrylate ou des adhésifs siliconés et un deuxième polymère ne pouvant pas ou ne pouvant que très peu être mélangé au polymère de base, avec une faible perméabilité à la vapeur d'eau, lequel est du polyisobutylène, un polymère séquencé styrène-isoprène-styrène ou un polymère séquencé styrène-butadiène-styrène, est dispersé dans au moins une des couches polymères, dans lequel la part de particules dispersées du deuxième polymère dans la matrice se situe entre 10 et 30 % en poids, dans lequel la masse surfacique de la matrice se situe entre 50 et 400 g/m².

2. Pansement transdermique ou topique selon la revendication 1, **caractérisé en ce que** la matrice est monocouche.

3. Pansement transdermique ou topique selon la revendication 1, **caractérisé en ce que** la phase, dispersée dans le polymère de base, du polymère avec une faible perméabilité à la vapeur d'eau présente une taille de particule moyenne de 5 à 50 µm, de préférence de 7 à 40 µm, en particulier de 10 à 30 µm.

4. Pansement transdermique ou topique selon l'une ou plusieurs des revendications 1 à 3, **caractérisé en ce que** la masse surfacique de la matrice se situe entre 60 et 300 g/m², en particulier entre 70 et 200 g/m².

5. Pansement transdermique ou topique selon l'une ou plusieurs des revendications 1 à 4, **caractérisé en ce que** sa perméabilité à la vapeur d'eau mesurée selon la méthode DIN EN 13726-2 pour une taille d'échantillon de 20 cm² à 37 °C et 18 % d'humidité relative se situe entre 50 et 600 g/(m² x 24 h), de préférence entre 100 et 500 g/(m² x 24 h), en particulier entre 150 et 400 g/(m² x 24 h).

6. Pansement transdermique ou topique selon l'une ou plusieurs des revendications 1 à 5, **caractérisé en ce que** le principe actif pharmaceutique est un anti-inflammatoire non stéroïdien.

7. Pansement transdermique ou topique selon la revendication 6, **caractérisé en ce que** le principe actif pharmaceutique est le diclofénac ou un de ses sets pharmaceutiquement acceptables, le kétoprofène, l'ibuprofène, le flurbiprofène, le naproxène, l'acide tiaprofénique, l'indométacine, le piroxicam, le ténoxicam, le méloxicam, l'acide flufénamique ou l'acide méfénamique.

8. Pansement transdermique ou topique selon la revendication 7, **caractérisé en ce que** le principe actif pharmaceutique est un sel de diclofénac, en particulier un sel de sodium de diclofénac, un sel de potassium de diclofénac, un sel de diéthylammonium de diclofénac ou le sel de pyrrolidine de dihydroxyéthyle de diclofénac.

9. Pansement transdermique ou topique selon l'une ou plusieurs des revendications précédentes 1 à 5, **caractérisé en ce que** le principe actif pharmaceutique est un antalgique à activité topique.

10. Pansement transdermique ou topique selon la revendication 9, **caractérisé en ce que** l'antalgique à activité topique est la lidocaïne ou la tétracaïne.

11. Pansement transdermique ou topique selon l'une ou plusieurs des revendications 1 à 10, **caractérisé en ce que** la matrice contient au moins un activateur de perméation, de préférence dans le polymère de base formant la structure.

12. Pansement transdermique ou topique selon la revendication 11, **caractérisé en ce que** le ou les activateurs de perméation sont des monoalcools ou polyalcools de bas poids moléculaire, des acides gras, de préférence de l'acide oléique, des alcools gras, des éthers d'alcool gras, des alcools gras polyoxyéthylés, des esters d'acide gras, des esters d'acide gras de sorbitan, des esters d'acide gras de sorbitan polyoxyéthylés et/ou de l'isosorbide de diméthyle.
